# EUROPEAN PATENT SPECIFICATION

(11) **EP 2 755 022 B1**
(45) Date of publication and mention of the grant of the patent: **30.05.2018**
(21) Application number: 13151378.0
(22) Date of filing: 15.01.2013
(51) Int. Cl.: G01N 33/487

(54) **Nanosensor**
Nanosensor
Nanocapteur

(43) Date of publication of application: 16.07.2014
(73) Proprietor: Honeywell International Inc., Morris Plains, NJ 07950 (US)
(72) Inventor: Mihaila, Mihai N., Morristown, NJ 07962-2245 (US); Serban, Bogdan, Morristown, NJ 07962-2245 (US); Costea, Stefan D., Morristown, NJ 07962-2245 (US)
(74) Representative: Houghton, Mark Phillip

(56) References cited:
- US-A1- 2004 229 386
- US-A1- 2010 327 847
- PING XIE ET AL: "Local electrical potential detection of DNA by nanowire-nanopore sensors", NATURE NANOTECHNOLOGY, vol. 7, no. 2, 11 December 2011 (2011-12-11), pages 119-125, XP055063150, ISSN: 1748-3387, DOI: 10.1038/nnano.2011.217
- ALEKSANDAR P. IVANOV ET AL: "DNA Tunneling Detector Embedded in a Nanopore", NANO LETTERS, vol. 11, no. 1, 12 January 2011 (2011-01-12), pages 279-285, XP055063157, ISSN: 1530-6984, DOI: 10.1021/nl103873a
- LIANG XIAOGAN ET AL: "Nanogap detector inside nanofluidic channel for fast real-time label-free DNA analysis", NANO LETTERS, AMERICAN CHEMICAL SOCIETY, US, vol. 8, no. 5, 1 May 2008 (2008-05-01), pages 1472-1476, XP002564344, ISSN: 1530-6984, DOI: 10.1021/NL080473K [retrieved on 2008-04-17]
- FERRY PRINS ET AL: "Room-Temperature Gating of Molecular Junctions Using Few-Layer Graphene Nanogap Electrodes", NANO LETTERS, vol. 11, no. 11, 9 November 2011 (2011-11-09), pages 4607-4611, XP055164484, ISSN: 1530-6984, DOI: 10.1021/nl202065x
- SEUNG KYU MIN ET AL: "Fast DNA sequencing with a graphene-based nanochannel device", NATURE NANOTECHNOLOGY, vol. 6, no. 3, 6 February 2011 (2011-02-06), pages 162-165, XP055164483, ISSN: 1748-3387, DOI: 10.1038/nnano.2010.283

## Description

### Technical Field

The present invention relates to a nanosensor.

### Background

Sensing (e.g., the detection of) dangerous molecules and/or organic volatile compounds may be performed in a number of fields, such as the safety and/or counter-terrorism fields, for example. In such fields, it may be important to be able to sense (e.g., detect) small quantities of molecules and/or compounds, such as a few molecules, or even a single (e.g., one) molecule. However, previous molecular sensors may not be capable of sensing such small quantities of molecules and/or compounds.
Ping et al:" Local Electrical Potential Detection of DNA by Nanowire Nanopore Sensors", Nature Technology, volume 7, 11 December 2011, pages 119-125 discloses that solid-state nanopores can be combined with silicon nanowire field-effect transistors to create sensors in which detection is localized and self-aligned at the nanopore.
US 2010/327847 discloses a solid state molecular sensor having an aperture extending through a thickness of a sensing region. The thickness of the sensing region corresponds to the characteristic extent of at least a component of a molecular species to be translocated through the aperture.
Aleksandar et al:" DNA tunnelling detector embedded in a nanopore", Nano letters, volume 11, 12 January 2011, pages 279-285 discloses a DNA nanopore detector with integrated tunneling electrodes. US2004/0229386 discloses a method for controlling the fabrication of nanogap of nanosensors.

The present invention in its various aspects is as set out in the appended claims.

### Brief Description of the Drawings

Figure 1 illustrates a nanosensor outside the scope of the present invention.
Figure 2 illustrates a nanosensor outside the scope of the present invention.
Figure 3 illustrates a nanosensor outside the scope of the present invention.
Figure 4 illustrates a nanosensor in accordance with the present invention.
Figure 5 is a graph illustrating the variation of the resistance of a nanogap of a nanosensor in accordance with with the present invention.

### Detailed Description

Nanosensors and methods of operating nanosensors are described herein.

Nanosensors can sense (e.g., detect) small quantities of molecules and/or compounds. For example, can sense a few molecules, or even a single (e.g., one) molecule.

In the following detailed description, reference is made to the accompanying drawings that form a part hereof.

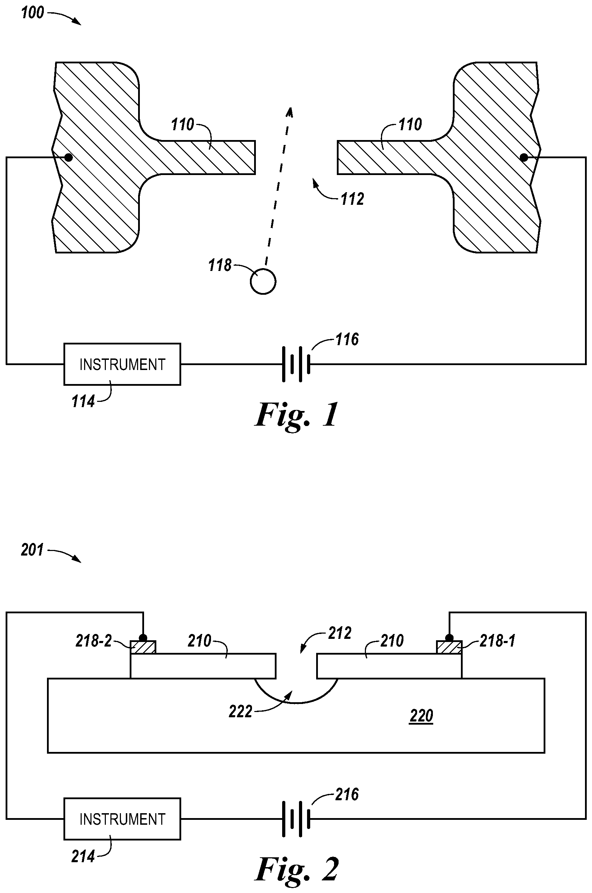

The figures herein follow a numbering convention in which the first digit or digits correspond to the drawing figure number and the remaining digits identify an element or component in the drawing. Similar elements or components between different figures may be identified by the use of similar digits. For example, 114 may reference element "14" in Figure 1, and a similar element may be referenced as 214 in Figure 2.

As used herein, "a" or "a number of" something can refer to one or more such things. For example, "a number of molecules" can refer to one or more molecules.

Figure 1 illustrates a nanosensor 100 outside the scope of the present invention. Nanosensor 100 can be, for example, a single molecule nanosensor, as will be further described herein.

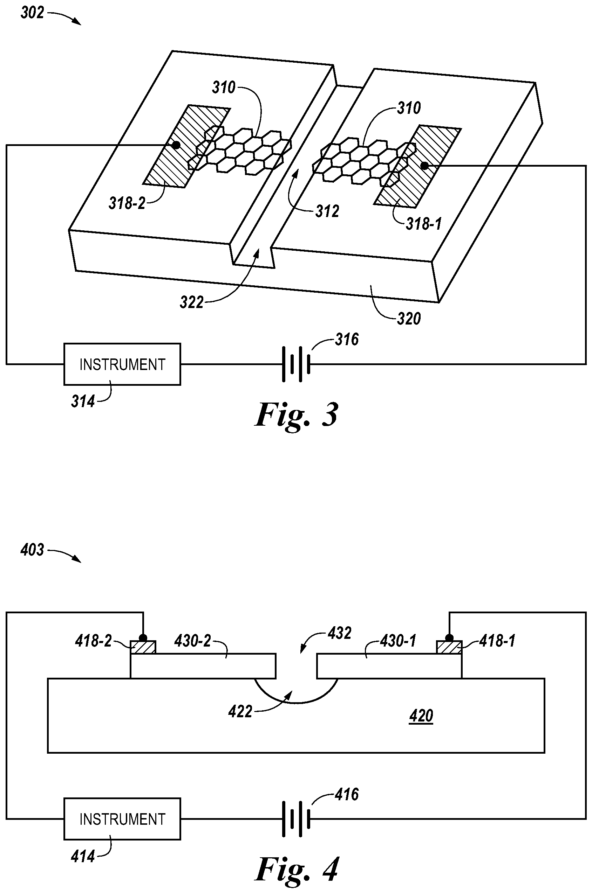

As shown in Figure 1, nanosensor 100 can include a nanomaterial 110. Nanomaterial 110 can be, for example, a conducting material, such as a semiconductor or a metal, among other conducting materials. In the embodiment illustrated in Figure 1, nanomaterial 110 is a nanowire. The nanowire can be, for example, a silicon nanowire. For example, nanomaterial 110 can be a nanoribbon, as will be further described herein (e.g., in connection with Figure 3).

As shown in Figure 1, nanosensor 100 can include a nanogap 112 in nanomaterial 110. Nanogap 112 can be formed in nanomaterial 110 by, for example, nanolithography, a focused ion beam, or electrically stressing nanomaterial 110, among other methods. As shown in Figure 1, nanogap 112 and a portion of nanomaterial 110 (e.g., a portion of nanomaterial 110 adjacent each side of nanogap 112) can be suspended such that a molecule(s) (e.g., molecule 118) can traverse (e.g., pass through) the nanogap, as will be further described herein. Nanogap 112 can be of a size (e.g., width) such that only a few molecules, or only a single (e.g., one) molecule (e.g., molecule 118), can traverse the nanogap at a time, as will be further described herein. That is, nanosensor 100 can be a single molecule nanosensor (e.g., nanosensor 100 can sense a single molecule at a time). For example, nanogap 112 can have a width of three nanometers or less. Nanogap can have a width of less than a nanometer. For example, nanogap 112 can be of a size such that more than a few molecules can traverse the nanogap at a time.

As shown in Figure 1, nanosensor 100 can include a voltage source 116 coupled to nanomaterial 110 (e.g., to a first portion of nanomaterial 110 that is on one side of nanogap 112 and to a second portion of nanomaterial 110 that is on the other side of nanogap 112). Voltage source 116 can apply a voltage to nanomaterial 110 (e.g., to the first and second portions of nanomaterial 110). When the voltage is applied to nanomaterial 110, a current (e.g., an electron current stream; not shown in Figure 1) may flow across nanogap 112. For a particular (e.g., known) voltage, the current across nanogap 112 can be characterized by (e.g., used to determine) the resistance of nanogap 112.

As shown in Figure 1, nanosensor 100 can include an instrument 114 in the circuit formed by voltage source 116 and nanomaterial 110. Instrument 114 can measure the current and/or resistance of nanogap 112 over time (e.g., while voltage source 116 is applying voltage to nanomaterial 110). For instance, instrument 114 can be an amperemeter (e.g., ammeter).

For example, as shown in Figure 1, a molecule (e.g., a single molecule) 118 can traverse (e.g., pass through) nanogap 112. While traversing nanogap 112, molecule 118 may modulate the current (e.g., scatter the electrons of the current) flowing across the nanogap, which may in turn modulate the resistance of the nanogap. For instance, the resistance of nanogap 112 while molecule 118 traverses the nanogap (e.g., the modulated resistance) may be a peak (e.g., maximum) resistance of the nanogap during the period of time in which instrument 114 is measuring the current and/or resistance of the nanogap. Further, the current and/or resistance of nanogap 112 while molecule 118 traverses the nanogap (e.g., the peak resistance of the nanogap) may depend on the type of the molecule. Accordingly, instrument 114 can measure the current and/or resistance of nanogap 112 while molecule 118 traverses the nanogap, and the molecule (e.g., the type of the molecule) can be identified based on the measured current and/or resistance (e.g., the peak intensity).

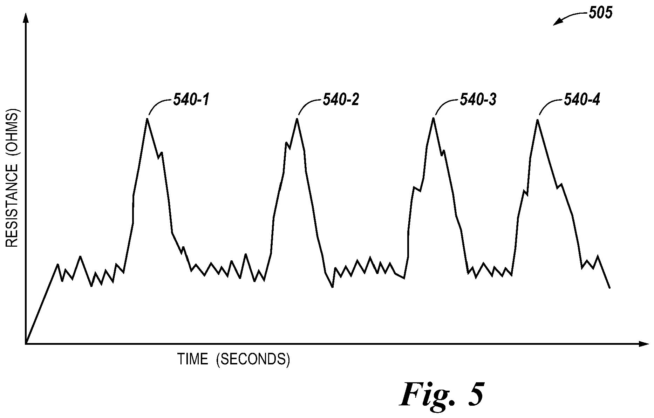

As an additional example, a number (e.g., a plurality) of molecules can sequentially traverse nanogap 112 (e.g., only one molecule may traverse nanogap 112 at a time). While traversing nanogap 112, each of the molecules may modulate the current and/or resistance of nanogap 112 in a manner analogous to molecule 118. For instance, the resistance of nanogap 112 while each molecule traverses the nanogap may be peak resistances of the nanogap during the period of time in which instrument 114 is measuring the current and/or resistance of the nanogap. Further, the current and/or resistance of nanogap 112 while each molecule traverses the nanogap (e.g., the peak resistances of the nanogap) may depend on the type of the molecule. For instance, if the molecules traversing nanogap 112 are the same type of molecule, the peak resistances may be approximately equal. Accordingly, instrument 114 can measure the current and/or resistance of nanogap 112 while each molecule traverses the nanogap, and the molecules can be identified based on the measured currents and/or resistances. Such an example will be further described herein (e.g., in connection with Figure 5).

Figure 2 illustrates a nanosensor 201 outside the scope of the present invention. Nanosensor 201 can be, for example, a single molecule nanosensor in a manner analogous to nanosensor 100 previously described in connection with Figure 1.

As shown in Figure 2, nanosensor 201 can include a nanomaterial 210. In the example illustrated in Figure 2, nanomaterial 210 is a nanowire. The nanowire can be, for example, a silicon nanowire. As an additional example, the nanowire can have a length of approximately 200 nanometers and/or a width (e.g., diameter) of approximately 15 nanometers.

As shown in Figure 2, nanosensor 201 can include a nanogap 212 in nanomaterial 210. Nanogap 212 can be formed in nanomaterial 210 by, for example, nanolithography, a focused ion beam, or electrically stressing nanomaterial 210, among other methods. Nanogap 212 can be of a size (e.g., width) such that only a few molecules, or only a single (e.g., one) molecule, can traverse the nanogap at a time, in a manner analogous to nanogap 112 previously described in connection with Figure 1. For example, nanogap 212 can be of a size such that more than a few molecules can traverse the nanogap at a time.

As shown in Figure 2, nanosensor 201 can include a substrate 220, with nanomaterial 210 formed on substrate 220. Substrate 220 can be, for example, an insulating substrate, such as silicon dioxide.

As shown in Figure 2, substrate 220 can have an opening (e.g., trench) 222 formed therein. Nanomaterial 210 can be formed on substrate 220 such that nanogap 212 and a portion of nanomaterial 210 (e.g., a portion of nanomaterial 210 adjacent each side of nanogap 212) can be suspended over opening 222, as illustrated in Figure 2. Accordingly, a molecule(s) can traverse (e.g., pass through) the nanogap, as will be further described herein.

As shown in Figure 2, nanosensor 201 can include metal contacts 218-1 and 218-2 coupled to nanomaterial 210. For example, metal contact 218-1 can be coupled to a first portion of nanomaterial 210 that is on one side of nanogap 212, and metal contact 218-2 can be coupled to a second portion of nanomaterial 210 that is on the other side of nanogap 212, as illustrated in Figure 2.

In the example illustrated in Figure 2, metal contacts 218-1 and 218-2 are coupled to the top of nanomaterial 210. For example, metal contacts 218-1 and/or 218-2 can be coupled to the side and/or bottom of nanomaterial 210.

Metal contacts 218-1 and 218-2 can be, for example, palladium contacts.

As shown in Figure 2, nanosensor 201 can include a voltage source 216 coupled to metal contacts 218-1 and 218-2 (e.g., voltage source 216 can be coupled to nanomaterial 210 via metal contacts 218-1 and 218-2). Voltage source 216 can apply a voltage to nanomaterial 210 (e.g., via metal contacts 218-1 and 218-2). When the voltage is applied to nanomaterial 210, a current (e.g., an electron current stream; not shown in Figure 2) may flow across nanogap 212. For a particular (e.g., known) voltage, the current across nanogap 212 can be characterized by (e.g., used to determine) the resistance of nanogap 212.

As shown in Figure 2, nanosensor 201 can include an instrument 214 in the circuit formed by voltage source 216 and nanomaterial 210. Instrument 214 can measure the current and/or resistance of nanogap 212 over time (e.g., while voltage source 216 is applying voltage to nanomaterial 210). For instance, instrument 214 can be an amperemeter (e.g., ammeter).

For example, a molecule (e.g., a single molecule) can traverse (e.g., pass through) nanogap 212. While traversing nanogap 212, the molecule may modulate the current (e.g., scatter the electrons of the current) flowing across the nanogap, which may in turn modulate the resistance of the nanogap, in a manner analogous to that previously described in connection with Figure 1. Accordingly, instrument 214 can measure the current and/or resistance of nanogap 212 while the molecule traverses the nanogap, and the molecule (e.g., the type of the molecule) can be identified based on the measured current and/or resistance, in a manner analogous to that previously described in connection with Figure 1.

As an additional example, a number (e.g., a plurality) of molecules can sequentially traverse nanogap 212 (e.g., only one molecule may traverse nanogap 212 at a time). While traversing nanogap 212, each of the molecules may modulate the current and/or resistance of nanogap 212 in a manner analogous to that previously described in connection with Figure 1. Accordingly, instrument 214 can measure the current and/or resistance of nanogap 212 while each molecule traverses the nanogap, and the molecules can be identified based on the measured currents and/or resistances, in a manner analogous to that previously described in connection with Figure 1. For instance, the variation of the resistance of nanogap 212 can be monitored, and the amplitude of the peak resistances randomly occurring can be used to identify the molecule.

Figure 3 illustrates a nanosensor 302 outside the scope of the present invention. Nanosensor 302 can be, for example, a single molecule nanosensor in a manner analogous to nanosensor 100 previously described in connection with Figure 1.

As shown in Figure 3, nanosensor 302 can include a nanomaterial 310. In the example illustrated in Figure 3, nanomaterial 310 is a nanoribbon. The nanoribbon can be, for example, a graphene nanoribbon.

As shown in Figure 3, nanosensor 302 can include a nanogap 312 in nanomaterial 310. Nanogap 312 can be formed in nanomaterial 310 by, for example, nanolithography, a focused ion beam, or electrically stressing nanomaterial 310, among other methods. Nanogap 312 can be of a size (e.g., width) such that only a few molecules, or only a single (e.g., one) molecule, can traverse the nanogap at a time, in a manner analogous to nanogap 112 previously described in connection with Figure 1. For example, nanogap 312 can be of a size such that more than a few molecules can traverse the nanogap at a time.

As shown in Figure 3, nanosensor 302 can include a substrate 320, with nanomaterial 310 formed on substrate 320. Substrate 320 can be analogous to substrate 220 previously described in connection with Figure 2. For example, as shown in Figure 3, substrate 320 can have an opening (e.g., trench) 322 formed therein, and nanomaterial 310 can be formed on substrate 320 such that nanogap 312 and a portion of nanomaterial 310 (e.g., a portion of nanomaterial 310 adjacent each side of nanogap 312) can be suspended over opening 322. Accordingly, a molecule(s) can traverse (e.g., pass through) the nanogap, as will be further described herein.

As shown in Figure 3, nanosensor 302 can include metal contacts 318-1 and 318-2 coupled to nanomaterial 310. For example, metal contact 318-1 can be coupled to a first portion of nanomaterial 310 that is on one side of nanogap 312, and metal contact 318-2 can be coupled to a second portion of nanomaterial 310 that is on the other side of nanogap 312, as illustrated in Figure 3. Further, metal contacts 318-1 and 318-2 can be coupled to the top or bottom of nanomaterial 310.

Metal contacts 318-1 and 318-2 can be, for example, palladium contacts.

As shown in Figure 3, nanosensor 302 can include a voltage source 316 coupled to metal contacts 318-1 and 318-2 (e.g., voltage source 316 can be coupled to nanomaterial 310 via metal contacts 318-1 and 318-2). Voltage source 316 can apply a voltage to nanomaterial 310 (e.g., via metal contacts 318-1 and 318-2). When the voltage is applied to nanomaterial 310, a current (e.g., an electron current stream; not shown in Figure 3) may flow across nanogap 312. For a particular (e.g., known) voltage, the current across nanogap 312 can be characterized by (e.g., used to determine) the resistance of nanogap 312.

As shown in Figure 3, nanosensor 302 can include an instrument 314 in the circuit formed by voltage source 316 and nanomaterial 310. Instrument 314 can measure the current and/or resistance of nanogap 312 over time (e.g., while voltage source 316 is applying voltage to nanomaterial 310). For instance, instrument 314 can be an amperemeter (e.g., ammeter).

For example, a molecule (e.g., a single molecule) can traverse (e.g., pass through) nanogap 312. While traversing nanogap 312, the molecule may modulate the current (e.g., scatter the electrons of the current) flowing across the nanogap, which may in turn modulate the resistance of the nanogap, in a manner analogous to that previously described in connection with Figure 1. Accordingly, instrument 314 can measure the current and/or resistance of nanogap 312 while the molecule traverses the nanogap, and the molecule (e.g., the type of the molecule) can be identified based on the measured current and/or resistance, in a manner analogous to that previously described in connection with Figure 1.

As an additional example, a number (e.g., a plurality) of molecules can sequentially traverse nanogap 312 (e.g., only one molecule may traverse nanogap 312 at a time). While traversing nanogap 312, each of the molecules may modulate the current and/or resistance of nanogap 312 in a manner analogous to that previously described in connection with Figure 1. Accordingly, instrument 314 can measure the current and/or resistance of nanogap 312 while each molecule traverses the nanogap, and the molecules can be identified based on the measured currents and/or resistances, in a manner analogous to that previously described in connection with Figure 1.

Figure 4 illustrates a nanosensor 403 according to the present invention. Nanosensor 403 can be, for example, a single molecule nanosensor in a manner analogous to nanosensor 100 previously described in connection with Figure 1.

As shown in Figure 4, nanosensor 403 includes two nanomaterials (e.g., separate nanomaterials) 430-1 and 430-2. Nanomaterials 430-1 and 430-2 can be, for example, conducting materials, such as semiconductors or metals, among other conducting materials. In the embodiment illustrated in Figure 4, nanomaterials 430-1 and 430-2 are nanowires. The nanowires can be, for example, silicon nanowires.

As shown in Figure 4, nanosensor 403 includes a nanogap 432 between nanomaterials 430-1 and 430-2. That is, nanogap 432 is formed using two separate nanomaterials (e.g., 430-1 and 430-2). According to the present invention, nanogap 432 is formed by prefabricating nanomaterials 430-1 and 430-2, and then placing nanomaterials 430-1 and 430-2 in the arrangement illustrated in Figure 4 (e.g., on substrate 420, as will be further described herein. Nanogap 432 can be of a size (e.g., width) such that only a few molecules, or only a single (e.g., one) molecule, can traverse the nanogap at a time, in a manner analogous to nanogap 112 previously described in connection with Figure 1. However, embodiments of the present disclosure are not limited to a particular size (e.g., width) for nanogap 432.

As shown in Figure 4, nanosensor 403 includes a substrate 420, with nanomaterials 430-1 and 430-2 formed on substrate 420. Substrate 420 can be analogous to substrate 220 previously described in connection with Figure 2. According to the present invention, as shown in Figure 4, substrate 420 has an opening (e.g., trench) 422 formed therein. Further, as shown in Figure 4, nanomaterials 430-1 and 430-2 are formed on substrate 420 such that nanogap 432, a portion of nanomaterial 430-1, and a portion of nanomaterial 430-2 (e.g., portions of nanomaterials 430-1 and 430-2 adjacent each side of nanogap 432) are suspended over opening 422. Accordingly, a molecule(s) can traverse (e.g., pass through) the nanogap, as will be further described herein.

As shown in Figure 4, nanosensor 403 includes metal contact 418-1 coupled to nanomaterial 430-1 and metal contact 418-2 coupled to nanomaterial 430-2. In the embodiment illustrated in Figure 4, metal contacts 418-1 and 418-2 are coupled to the tops of nanomaterials 430-1 and 430-2, respectively. However, embodiments of the present disclosure are not so limited. For example, in some embodiments, metal contacts 418-1 and/or 418-2 can be coupled to the side and/or bottom of nanomaterials 430-1 and/or 430-2, respectively.

Metal contacts 418-1 and 418-2 can be, for example, palladium contacts. However, embodiments of the present invention are not limited to a particular type of metal contact.

As shown in Figure 4, nanosensor 403 includes a voltage source 416 coupled to metal contacts 418-1 and 418-2 (e.g., voltage source 416 is coupled to nanomaterials 430-1 and 430-2 via metal contacts 418-1 and 418-2, respectively). Voltage source 416 applies a voltage to nanomaterials 430-1 and 430-2 (e.g., via metal contacts 418-1 and 418-2, respectively). When the voltage is applied to nanomaterials 430-1 and 430-2, a current (e.g., an electron current stream; not shown in Figure 4) may flow across nanogap 432. For a particular (e.g., known) voltage, the current across nanogap 432 can be characterized by (e.g., used to determine) the resistance of nanogap 432.

As shown in Figure 4, nanosensor 403 includes an instrument 414 in the circuit formed by voltage source 416 and nanomaterials 430-1 and 430-2. Instrument 414 measures the current and/or resistance of nanogap 432 over time (e.g., while voltage source 416 is applying voltage to nanomaterials 430-1 and 430-2). For instance, instrument 414 can be an amperemeter (e.g., ammeter).

For example, a molecule (e.g., a single molecule) can traverse (e.g., pass through) nanogap 432. While traversing nanogap 432, the molecule may modulate the current (e.g., scatter the electrons of the current) flowing across the nanogap, which may in turn modulate the resistance of the nanogap, in a manner analogous to that previously described in connection with Figure 1. Accordingly, instrument 414 can measure the current and/or resistance of nanogap 432 while the molecule traverses the nanogap, and the molecule (e.g., the type of the molecule) can be identified based on the measured current and/or resistance, in a manner analogous to that previously described in connection with Figure 1.

As an additional example, a number (e.g., a plurality) of molecules can sequentially traverse nanogap 432 (e.g., only one molecule may traverse nanogap 432 at a time). While traversing nanogap 432, each of the molecules may modulate the current and/or resistance of nanogap 432 in a manner analogous to that previously described in connection with Figure 1. Accordingly, instrument 414 can measure the current and/or resistance of nanogap 432 while each molecule traverses the nanogap, and the molecules can be identified based on the measured currents and/or resistances, in a manner analogous to that previously described in connection with Figure 1.

Figure 5 is a graph 505 illustrating the variation (e.g., time dependence) of the resistance of a nanogap of a nanosensor in accordance with the present invention over a period of time. The nanogap of the nanosensor can be, for example, nanogap 112 of nanosensor 100, nanogap 212 of nanosensor 201, nanogap 312 of nanosensor 302, and/or nanogap 432 of nanosensor 403 previously described herein in connection with Figures 1, 2, 3, and 4, respectively, and the resistance of the nanogap over the period of time can be measured by, for example, instrument 114, 214, 314, and/or 414 previously described in connection with Figures 1, 2, 3, and 4, respectively.

During the period of time illustrated in graph 505, a number of (e.g., four) molecules have sequentially traversed the nanogap of the nanosensor. While traversing the nanogap, each of the four molecules modulated the resistance of the nanogap in a manner analogous to that previously described in connection with Figure 1. Accordingly, peak (e.g., maximum) resistances 540-1, 540-2, 540-3, and 540-4 of graph 505 correspond to the resistance (e.g., the modulated resistance) of the nanogap when each molecule traversed the nanogap (e.g., peak resistance 540-1 corresponds to the resistance of the nanogap when the first molecule traversed the nanogap, peak resistance 540-2 corresponds to the resistance of the nanogap when the second molecule traversed the nanogap, etc.).

The values of peak resistances 540-1, 540-2, 540-3, and 540-4 can depend on the type of molecule traversing the nanogap of the nanosensor, as previously described herein (e.g., in connection with Figure 1). Further, as illustrated in graph 505, the values of peak resistances 540-1, 540-2, 540-3, and 540-4 are approximately equal. Accordingly, it can be determined that the molecules that have traversed the nanogap are of the same type, and the molecules (e.g., the type of the molecules) can be identified based on the values of peak resistances 540-1, 540-2, 540-3, and 540-4, as previously described herein (e.g., in connection with Figure 1).

## Claims

1. A nanosensor (403), comprising:
a substrate (420) having an opening (422) formed therein;
two separate nanomaterials (430-1, 430-2) formed on the substrate (420);
a nanogap (432) formed between the two separate nanomaterials (430-1, 430-2) such that the nanogap (432) and a portion of each of the two separate nanomaterials (430-1, 430-2) are suspended over the opening (422), **characterised in that** the nanogap (432) is formed by:
prefabricating the two separate nanomaterials (430-1, 430-2); and
placing the two separate nanomaterials (430-1, 430-2) on the substrate (420);
a voltage source 416, wherein the voltage source (416) is coupled to one of the two separate nanomaterials (430-1) that is on a first side of the nanogap (432) and the other one of the two separate nanomaterials (430-2) that is on a second side of the nanogap (412); and
an instrument (414) configured to:
measure a resistance of the nanogap (432) over a period of time, wherein:
a molecule (118) traverses the nanogap (432) during the period of time; and
the resistance of the nanogap (432) while the molecule (118) traverses the nanogap (432) is a maximum resistance during the period of time; and
identify a type of the molecule (118) based on the maximum resistance during the period of time.

2. The nanosensor (403) of claim 1, wherein the two separate nanomaterials (430-1, 430-2) are nanowires.

3. The nanosensor (403) of claim 2, wherein the nanowires are silicon nanowires.

4. The nanosensor (403) of claim 1, wherein the substrate (420) is silicon dioxide.

5. The nanosensor (403) of claim 1, wherein the nanogap (432) is of a size such that only a single molecule (118) can traverse the nanogap (432) at a time.

6. The nanosensor (403) of claim 1, wherein the nanosensor (403) includes:
a first metal contact (418-1) coupled to one of the two separate nanomaterials (430-1); and
a second metal contact (418-2) coupled to the other one of the two separate nanomaterials (430-2); and
the voltage source (416) is coupled to the first metal contact (418-1) and the second metal contact (418-2).

## Patentansprüche

1. Nanosensor (403), umfassend:
ein Substrat (420), das eine Öffnung (422) aufweist, die darin gebildet ist;
zwei getrennte Nanomaterialien (430-1, 430-2), die auf dem Substrat (420) gebildet sind;
einen Nanospalt (432), der zwischen den beiden getrennten Nanomaterialien (430-1, 430-2) so gebildet ist, dass der Nanospalt (432) und ein Teil von jedem der getrennten Nanomaterialien (430-1, 430-2) über der Öffnung (422) aufgehängt sind, **dadurch gekennzeichnet, dass** der Nanospalt (432) gebildet wird durch:
Vorfertigen der zwei getrennten Nanomaterialien (430-1, 430-2); und
Platzieren der zwei getrennten Nanomaterialien (430-1, 430-2) auf dem Substrat (420);
eine Spannungsquelle (416), wobei die Spannungsquelle (416) mit Einem der beiden getrennten Nanomaterialien (430-1), das sich auf einer ersten Seite des Nanospalts (432) befindet, und mit dem Anderen der beiden getrennten Nanomaterialien (430-2) verbunden ist, das sich auf einer zweiten Seite des Nanospalts (412) befindet; und
ein Instrument (414), das konfiguriert ist zum:
Messen eines Widerstands des Nanospalts (432) über einen Zeitraum, wobei:
ein Molekül (118) den Nanospalt (432) während des Zeitraums durchquert; und
der Widerstand des Nanospalts (432) ein maximaler Widerstand während des Zeitraums ist, in dem das Molekül (118) den Nanospalt (432) durchquert; und
Identifizieren einer Art des Moleküls (118) aufgrund des maximalen Widerstands während des Zeitraums.

2. Nanosensor (403) nach Anspruch 1, wobei die zwei getrennten Nanomaterialien (430-1, 430-2) Nanodrähte sind.

3. Nanosensor (403) nach Anspruch 2, wobei die Nanodrähte Siliziumnanodrähte sind.

4. Nanosensor (403) nach Anspruch 1, wobei das Substrat (420) ein Siliziumdioxid ist.

5. Nanosensor (403) nach Anspruch 1, wobei der Nanospalt (432) eine Größe aufweist, sodass zu einem Zeitpunkt jeweils nur ein einziges Molekül (118) den Nanospalt (432) durchqueren kann.

6. Nanosensor (403) nach Anspruch 1, wobei der Nanosensor (403) umfasst:
einen ersten Metallkontakt (418-1), der mit Einem der beiden getrennten Nanomaterialien (430-1) verbunden ist; und
einen zweiten Metallkontakt (418-2), der mit dem Anderen der beiden getrennten Nanomaterialien (430-2) verbunden ist; und
wobei die Spannungsquelle (416) mit dem ersten Metallkontakt (418-1) und dem zweiten Metallkontakt (418-2) verbunden ist.

## Revendications

1. Nanocapteur (403) comportant :
un substrat (420) dans lequel est formée une ouverture (422) ;
deux nanomatériaux distincts (430-1, 430-2) formés sur le substrat (420) ;
un nano-écartement (432) formé entre les deux nanomatériaux distincts (430-1, 430-2) de telle façon que le nano-écartement (432) et une partie de chacun des deux nanomatériaux distincts (430-1, 430-2) soient suspendus au-dessus de l'ouverture (422), **caractérisé en ce que** le nano-écartement (432) est formé en :
pré-fabriquant les deux nanomatériaux distincts (430-1, 430-2) ; et
en plaçant les deux nanomatériaux distincts (430-1, 430-2) sur le substrat (420) ;
une source (416) de tension, la source (416) de tension étant couplée à un des deux nanomatériaux distincts (430-1) qui se trouve d'un premier côté du nano-écartement (432) et à l'autre des deux nanomatériaux distincts (430-2) qui se trouve d'un deuxième côté du nano-écartement (412) ; et
un instrument (414) configuré pour :
mesurer une résistance du nano-écartement (432) sur un laps de temps :
une molécule (118) traversant le nano-écartement (432) pendant le laps de temps ; et
la résistance du nano-écartement (432) tandis que la molécule (118) traverse le nano-écartement (432) étant une résistance maximale pendant le laps de temps ; et
identifier un type de la molécule (118) d'après la résistance maximale pendant le laps de temps.

2. Nanocapteur (403) selon la revendication 1, les deux nanomatériaux distincts (430-1, 430- 2) étant des nanofils.

3. Nanocapteur (403) selon la revendication 2, les nanofils étant des nanofils en silicium.

4. Nanocapteur (403) selon la revendication 1, le substrat (420) étant du dioxyde de silicium.

5. Nanocapteur (403) selon la revendication 1, le nano-écartement (432) étant d'une taille telle qu'une seule molécule (118) à la fois puisse traverser le nano-écartement (432).

6. Nanocapteur (403) selon la revendication 1, le nanocapteur (403) comprenant :
un premier contact métallique (418-1) couplé à un des deux nanomatériaux distincts (430-1) ; et
un deuxième contact métallique (418-2) couplé à l'autre des deux nanomatériaux distincts (430-2) ; et
la source (416) de tension étant couplée au premier contact métallique (418-1) et au deuxième contact métallique (418-2).
